# EUROPEAN PATENT SPECIFICATION

(11) **EP 2 877 838 B1**
(45) Date of publication and mention of the grant of the patent: **11.11.2020**
(21) Application number: 13822410.0
(22) Date of filing: 24.07.2013
(51) Int. Cl.: G01N 21/64, G01N 21/55, H04W 88/02

(54) **IMMUNOASSAY RAPID DIAGNOSTIC TEST UNIVERSAL ANALYSIS DEVICE, SYSTEM, METHOD AND COMPUTER READABLE MEDIUM**
VORRICHTUNG, VERFAHREN UND SYSTEM FÜR UNIVERSELLE ANALYSEN IN SCHNELLEN IMMUNASSAY-DIAGNOSETESTS UND COMPUTERLESBARES MEDIUM
DISPOSITIF, SYSTÈME, PROCÉDÉ ET SUPPORT APTE À ÊTRE LU PAR ORDINATEUR POUR ANALYSE UNIVERSELLE D'ESSAI DE DIAGNOSTIC RAPIDE D'IMMUNODOSAGE

(30) Priority: 24.07.2012 US 201261675049 P
(43) Date of publication of application: 03.06.2015
(73) Proprietor: Fio Corporation, Toronto, ON M5C 1S2 (CA)
(72) Inventor: XIANG, Qing, Toronto, ON M2M 2K3 (CA); LEE, Jeongjin, Vaughan, ON L6A 1W7 (CA)
(74) Representative: Forresters IP LLP
(86) International application number: PCT/CA2013/000670
(87) International publication number: WO 2014/015420

(56) References cited:
- WO-A1-2010/081219
- WO-A1-2012/012499
- WO-A2-03/001889
- WO-A2-2007/053186
- US-A- 5 408 535
- US-A1- 2007 196 095
- US-A1- 2012 071 342
- US-A1- 2012 123 686
- ONUR MUDANYALI ET AL: "Integrated rapid-diagnostic-test reader platform on a cellphone", LAB ON A CHIP, vol. 12, no. 15, 1 January 2012 (2012-01-01), pages 2678-2686, XP055058669, ISSN: 1473-0197, DOI: 10.1039/c2lc40235a

## Description

### FIELD OF THE INVENTION

The present invention relates generally to an rapid diagnostic test analysis device and method, and more particularly to an immunoassay rapid diagnostic test universal analysis device, system, method and computer readable medium.

### BACKGROUND OF THE INVENTION

In the prior art, the rapid immunoassay diagnostic test may have achieved great commercial application in many fields - potentially including, for example, in association with pregnancy tests, infectious disease tests, oncology tests, cardiovascular disease tests, animal health tests, and/or tests related to foods and the environment, etc. One of the most successful examples may be all kinds of lateral flow immunoassay tests.

Usually, lateral flow types of rapid tests may have been visually interpreted by end users. Such visual interpretation by end users may have been subject to inconsistency and/or human interference. There may be a trend to using devices to analyze the results of such tests. There may be a number of US patents and/or published US patent applications which may have disclosed methods and/or devices to automatically analyze lateral flow tests. There may be a great amount of diversity in the various prior art lateral flow cassettes, whether by application, manufacture, and/or signal format. It may have been almost impossible for a device to read more than one type of these products. Typically, prior art devices for reading lateral flow tests may have been adapted to read just one specific lateral flow product and/or test.

Even were it not so, prior art devices may have been adapted to just read only a single type of test - i.e., either reflection signals (e.g., from a colloidal gold and/or colored latex bead type of test) or emission signal (e.g., from a fluorescence type of test).

Furthermore, prior art devices manufactured by any particular company may have been adapted just to read test products which were also manufactured by that particular company. In order to read a different product, such devices would have needed to be customized and/or reconfigured based on differences in physical appearance, detection areas, control lines and test line positions in the various products. Such an endeavor might have been highly unlikely to have been performed, if at all.

The present invention may preferably, but need not necessarily, provide a method, system and/or device to universally analyze various immunoassay rapid diagnostic tests.

The present invention may preferably address, mitigate, alleviate, and/or overcome one or more of the aforementioned disadvantages, shortcomings, problems and/or other issues associated with the prior art, and/or to achieve one or more of the aforementioned objects of the invention.

WO 2012/012499 A1 relates to an optical reader system for determining the levels of analytes present in a sample that is presented on a cassette. WO 2007/053186A2 relates to a portable optical reader system for analyzing blood components present in a sample that is presented on a cassette. Also relevant to the technical field of the present invention is Onur Mudanyali et al: "Integrated rapid-diagnostic-test reader platform on a cellphone", Lab on a chip, vol. 12, no. 15, 1 January 2012, pages 2678-2686.

### SUMMARY OF THE INVENTION

According to the invention, there is disclosed a device for analysis of various types of cassettes for immunoassay rapid diagnostic tests, as set out in claim 1.

According to an aspect of one preferred embodiment of the invention, the processor may preferably, but need not necessarily, automatically analyze the test signal, with reference to the applicable test protocol for the loaded cassette.

According to an aspect of one preferred embodiment of the invention, the test signal may preferably, but need not necessarily, include a test line signal, corresponding to a test line present on the loaded cassette after incubation. The processor automatically measures an intensity of the test line signal.

According to an aspect of one preferred embodiment of the invention, the test signal may preferably, but need not necessarily, include a control line signal corresponding to a control line present on the loaded cassette after incubation. The processor automatically measures an intensity of the control line signal.

According to an aspect of one preferred embodiment of the invention, the applicable test protocol may preferably, but need not necessarily, include a predetermined assay threshold value. The processor analyzes the test signal with reference to the assay threshold value to automatically determine a diagnostic test result associated with the loaded cassette.

According to an aspect of one preferred embodiment of the invention, the device may preferably, but need not necessarily, include an output device which automatically presents the diagnostic test result to a user of the device.

According to an aspect of one preferred embodiment of the invention, the device may preferably, but need not necessarily, include a memory onboard the device which stores one or more sets of executable instructions, preferably to encode the processor to automatically analyze the test signal as aforesaid.

According to an aspect of one preferred embodiment of the invention, the device may preferably, but need not necessarily, include a memory onboard the device which may but need not necessarily, store one or more sets of executable instructions, preferably to encode the processor to automatically identify the applicable test protocol as aforesaid.

According to an aspect of one preferred embodiment of the invention, the device may preferably, but need not necessarily, include a memory onboard the device which stores the database.

According to an aspect of one preferred embodiment of the invention, the device may preferably, but need not necessarily, include a communications element onboard the device which the processor automatically uses to remotely reference the database as aforesaid.

According to an aspect of one preferred embodiment of the invention, the device may preferably, but need not necessarily, include at least one optical filter. The processor automatically ensures the optical filter is moved to an engaged position between the loaded cassette and the imaging element before lighting a corresponding emission LED. The imaging element then captures the test signal through the optical filter. The processor automatically ensures the optical filter is moved to a disengaged position clear of the imaging element before lighting the reflection LED. The imaging element then captures the test signal clear of the optical filter.

According to an aspect of one preferred embodiment of the invention, the optical filter may preferably, but need not necessarily, be mounted on a sliding switch which slides the optical filter between the engaged position and the disengaged position.

According to an aspect of one preferred embodiment of the invention, the optical filter may preferably, but need not necessarily, be mounted on a rotatable mechanism which rotates the optical filter between the engaged position and the disengaged position.

According to an aspect of one preferred embodiment of the invention, the optical filter may preferably, but need not necessarily, be an optical band pass filter.

According to an aspect of one preferred embodiment of the invention, the device may preferably, but need not necessarily, include an optical long pass filter positioned between the loaded cassette and the imaging element. The imaging captures the test signal through the optical long pass filter.

According to an aspect of one preferred embodiment of the invention, the aforesaid at least one reflection LED may preferably, but need not necessarily, include at least one white LED.

According to an aspect of one preferred embodiment of the invention, the aforesaid at least one fluorescent LED may preferably, but need not necessarily, include at least one ultraviolet LED.

According to an aspect of one preferred embodiment of the invention, the aforesaid at least one fluorescent LED may preferably, but need not necessarily, include at least one colored LED.

According to an aspect of one preferred embodiment of the invention, the processor may preferably, but need not necessarily, identify the applicable test protocol with reference to one or more of the following which are captured in the first image stored in the database for the loaded cassette: one or more cassette dimensions; one or more cassette shapes; one or more detection line dimensions; one or more control line dimensions; one or more detection areas; one or more membrane areas; one or more control line positions; one or more test line positions; one or more cassette colors; one or more line colors; manufacturer indicia; product indicia; brand name indicia; application indicia; disease indicia; test type indicia; incubation time indicia; expected results indicia; barcodes; two-dimensional barcodes; labels; and/or other printed and written indicia.

According to an aspect of one preferred embodiment of the invention, the processor may preferably, but need not necessarily, additionally identify the applicable test protocol with reference to one or more of the following which are received by the device from the loaded cassette and stored in the database for the loaded cassette: magnetically stored data, fluorescence data, and/or radioactive signal data.

According to an aspect of one preferred embodiment of the invention, the device may preferably, but need not necessarily, be adapted for analysis of various lateral flow cassettes as the test cassettes.

According to an aspect of one preferred embodiment of the invention, the device may preferably, but need not necessarily, be adapted for use with a cellular telephone to provide at least one of the imaging element and the processor.

According to an aspect of one preferred embodiment of the invention, left and right channel audio signals from the cellular telephone are adapted to turn the reflection LED and the fluorescent LED on and/or off.

According to an aspect of one preferred embodiment of the invention, the imaging element may preferably, but need not necessarily, include one or more scanning heads.

According to the invention, there is also disclosed a system for analysis of various types of cassettes for immunoassay rapid diagnostic tests, as set out in claim 15.

According to an aspect of one preferred embodiment of the invention, left and/or right channel audio signals from the cellular telephone are adapted to turn the reflection LED and the fluorescent LED on and off.

According to the invention, there is also disclosed a method for analysis of various types of cassettes for immunoassay rapid diagnostic tests, as set out in claim 16.

According to an aspect of one preferred embodiment of the invention the processor automatically analyzes the test signal with reference to the applicable test protocol for the loaded cassette.

The test signal comprises a test line signal corresponding to a test line present on the loaded cassette after incubation. The processor automatically measures an intensity of the test line signal.

According to an aspect of one preferred embodiment of the invention, in the processing step, the test signal comprises a control line signal a test line signal corresponding to a control line present on the loaded cassette after incubation. The processor automatically measures an intensity of the control line signal.

According to an aspect of one preferred embodiment of the invention, in the processing step, the applicable test protocol may preferably, but need not necessarily, include a predetermined assay threshold value. The processor analyzes the test signal with reference to the assay threshold value to automatically determine a diagnostic test result associated with the loaded cassette.

According to an aspect of one preferred embodiment of the invention, the method may preferably, but need not necessarily, include a presentation step, wherein the diagnostic test result is automatically presented using an output device.

According to an aspect of one preferred embodiment of the invention, preferably before the processing step, one or more sets of executable instructions are stored in a memory. Also in the processing step, the executable instructions encode the processor to automatically analyze the test signal as aforesaid.

According to an aspect of one preferred embodiment of the invention, preferably before the processing step, one or more sets of executable instructions are stored in a memory. Also in the processing step, the executable instructions encode the processor to automatically identify the applicable test protocol as aforesaid.

According to an aspect of one preferred embodiment of the invention, preferably in the processing step, the processor automatically uses a communications element, preferably to remotely reference the database as aforesaid.

According to an aspect of one preferred embodiment of the invention, the method may preferably, but need not necessarily, also include a filtering step of providing at least one optical filter. In the processing step, (i) the processor automatically ensures the optical filter is moved to an engaged position between the loaded cassette and the imaging element before lighting a corresponding emission LED. The imaging element then captures the test signal through the optical filter. In the processing step, (ii) the processor automatically ensures the optical filter is moved to a disengaged position clear of the imaging element before lighting the reflection LED. The imaging element then captures the test signal clear of the optical filter.

According to an aspect of one preferred embodiment of the invention, the method may preferably, but need not necessarily, also include a filtering step of providing an optical long pass filter positioned between the loaded cassette and the imaging element. In the processing step, the imaging element captures the test signal through the optical long pass filter.

According to an aspect of one preferred embodiment of the invention, preferably in the LED step, at least one white LED is provided as the aforesaid at least one reflection LED.

According to an aspect of one preferred embodiment of the invention, preferably in the LED step, at least one ultraviolet LED is provided as the aforesaid at least one fluorescent LED.

According to an aspect of one preferred embodiment of the invention, preferably in the LED step, at least one colored LED is provided as the aforesaid at least one fluorescent LED.

According to an aspect of one preferred embodiment of the invention, preferably in the processing step, the processor identifies the applicable test protocol with reference to one or more of the following which are captured in the first image and stored in the database for the loaded cassette: one or more cassette dimensions; one or more cassette shapes; one or more detection line dimensions; one or more control line dimensions; one or more detection areas; one or more membrane areas; one or more control line positions; one or more test line positions; one or more cassette colors; one or more line colors; manufacturer indicia; product indicia; brand name indicia; application indicia; disease indicia; test type indicia; incubation time indicia; expected results indicia; barcodes; two-dimensional barcodes; labels; and other printed and written indicia.

According to an aspect of one preferred embodiment of the invention, preferably in the processing step, the processor additionally identifies the applicable test protocol with reference to one or more of the following which are received from the loaded cassette and stored in the database for the loaded cassette: magnetically stored data; fluorescence data; and radioactive signal data.

According to an aspect of one preferred embodiment of the invention, preferably at least in the imaging step and the processing step, a cellular telephone is provided as the aforesaid imaging element and/or as the aforesaid processor.

According to an aspect of one preferred embodiment of the invention, preferably at least in the imaging step and the processing step, left and right channel audio signals from the cellular telephone turn the reflection LED and the fluorescent LED on and off.

In the imaging step, one or more scanning heads are provided as at least part of the aforesaid imaging element.

According to the invention, there is also disclosed a computer readable medium for analysis of various types of cassettes for immunoassay rapid diagnostic tests, configured to be used with the device of claim 1. The computer readable medium includes executable instructions, which are physically stored thereon. The instructions, upon execution, encode at least one processor to automatically capture a first image of the loaded cassette using the imaging element when at least one of the LEDs is lit. The instructions, upon execution, encode the aforesaid at least one processor to automatically identify, using the first image and the database, an applicable test protocol for the loaded cassette. The instructions, upon execution, encode the aforesaid at least one processor to automatically, after incubation of the loaded cassette and depending on the applicable one said test protocol: (i) determine if the first image captures a post-incubation test signal from the loaded cartridge; and otherwise (ii) light the fluorescent LED to generate the test signal by emission, and the reflection LED to generate the test signal by reflection, from the loaded cassette and uses the imaging element to capture a second image of the loaded cassette and of the test signal. The instructions, upon execution, encode the aforesaid at least one processor to automatically provide the test signal, in the first image or the second image, for analysis of the loaded cassette.

Also described is a device, system and/or method which may be configured for use in association with one or more lateral flow product databases.

These databases, and/or any others provided and/or called for according to the invention, may preferably, but need not necessarily, be local to the device, to other components of the system, and/or to the computer readable medium, and/or they may preferably, but need not necessarily, be remote therefrom, preferably with a communications element being used to remotely access and/or reference them. According to the invention, the databases may may preferably, but need not necessarily, take the form of one or more local, remote, distributed, congruent and/or peer-to-peer databases, which may preferably, but need not necessarily, be accessible by the device locally and/or over one or more of its regular wireless (and/or wired) communication networks, including terrestrial and/or satellite networks -- e.g., the Internet and/or cloud-based networks.

Preferably, the databases may comprise, for each lateral flow product, a collection of data about manufacturer information, product information, test type, identification information, incubation time, and/or expected results, etc. Manufacturer information stored in the databases may preferably comprise information concerning the manufacturer of the product. Product information stored in the databases may preferably comprise information concerning product brand names and/or application information. Identification data stored in the databases may preferably comprise specific data which can be used to discriminate the individual product from others, and/or which can be used to identify the test by the device, system and/or method according to the present invention. Specific data may include data concerning one or more physical dimensions and/or colors associated with the product, and/or it may include and/or be encoded in writing and/or prior art labeling techniques (and/or other indicia, labels, writing, printing, markings, text, characters and/or symbols, with these terms understood by persons skilled in the art to be capable of being used interchangeably *mutatis mutandis* herein) which may be associated with a particular product and/or test, such as, for example, barcode images, magnetically stored data, fluorescence data, and/or radioactive signal data, etc.

As described, test identification may preferably be performed in a process by which the device, system and/or method may preferably automatically recognize a cassette type and/or its manufacturer. Preferably, a pre-defined assay cut-off value (e.g., a positive and/or negative threshold) may be applied for that type of cassette for diagnostic purposes.

As described, system and/or method may preferably, but need not necessarily, include an electronic board, a processor (e.g., a microprocessor), image analysis and/or processing software, a light chamber, one or more white (cassette recognition, reflection) LEDs, one or more color (emission) LEDs and/or ultraviolet ("UV") LEDs, an optical filter, and/or a color camera. Preferably, the optical filter may be an optical band pass filter and/or an optical long pass filter. Preferably, the optical filter may be mounted on a sliding switch and/or on a rotatable mechanism, and/or it may be moved towards and/or away from the front of the camera. Preferably, the LEDs may be illuminated (i.e., on) to permit the camera to take a picture, and may preferably just illuminate the light chamber when the camera is taking a picture. The processor and/or controlling electronics may control whether one or more of the LEDs is on or off. The device, system and/or method may preferably enable one or more lateral flow cassettes to be received for recognition and analysis. The microprocessor may preferably be adapted with image processing software for analysis of one or more images of the lateral flow cassettes.

As described, for reading reflection signal type of tests (e.g., colloidal gold and/or colored latex bead types of tests), the white LEDs may preferably be on. [References herein to use of the invention with colloidal gold tests and/or colored latex bead tests may be considered, more generally, as references to its use (if and when appropriate) with reflection signal tests, with any changes which may be necessary and/or appropriate for such use.] In such circumstances, the color and/or UV LEDs may preferably be off. The optical filter may preferably be switched away from the color camera. Preferably, the color camera may take an image of the entire cassette, preferably including the plastic case and/or membrane area.

As described, the image taken by the camera may preferably be analyzed by the image analysis and/or processing software in the microprocessor. [Each reference herein to image analysis software and/or to image processing software may be considered as a reference (if and when appropriate) to the other, to imaging software, and/or to software more generally, with any changes which may be necessary and/or appropriate in such instance.] The function of the image analysis software may preferably include: (1) cassette recognition, preferably with reference to cassette features (such as for example size, aspect ratio, color, shape, letters, etc.) and/or so as to provide a user with information concerning the cassette manufacturer, disease to be tested, etc.; (2) membrane region identification; (3) measurement of the intensity of the test line and/or control line in the membrane region and/or area; and/or (4) presenting diagnostic results according to one or more cut-off values, preferably as pre-set in the software.

The image processing software may preferably assess one or more images to determine and/or present the user cassette type and the diagnostic results to a user of the device, system and/or method according to the invention.

As described, for reading emission signal type of tests (e.g., fluorescent lateral flow cassette tests), two or more images may preferably but need not necessarily be taken for each test. [References herein to use of the invention with fluorescent signal tests may be considered, more generally, as references to its use (if and when appropriate) with emission signal tests, with any changes which may be necessary and/or appropriate for such use.] In such circumstances, the first image may preferably be an image of the cassette as taken with the white LED on, and the color LEDs off, and with the optical filter (e.g., an optical band pass filter) moved away from the camera. [Each reference herein to an optical band pass filter and/or to an optical long pass filter may be considered as a reference (if and when appropriate) to optical filters, more generally, and/or to another specific type of optical filter, with any changes which may be necessary and/or appropriate for such filter.] Preferably, after the first image, a further image (e.g., the second image) may be a fluorescent signal image of the membrane region, preferably with the color LEDs on and/or the white LEDs off, and preferably with the optical filter (e.g., the optical band pass filter) in front of the camera. Preferably, for emission signal tests, there may be no line visible in the membrane area in the first image because, preferably, the color and/or UV LEDs (which may be required for fluorescent lateral flow images) may be off. [Each reference herein to color LEDs and/or to UV LEDs may be considered as a reference (if and when appropriate) to the other and/or to non-white LEDs, more generally, with any changes which may be necessary and/or appropriate for such LEDs.] The first image may preferably enable software according to the invention to perform cassette recognition. Preferably, the fluorescent control line and/or test signal line may be present and/or visible in the second image. [According to one aspect of the present invention, the fluorescent control line and/or test signal line may be rendered visible by the optical filter.] The image processing software may preferably combine and/or assess the two or more images to determine and/or present the user cassette type and the diagnostic results to a user of the device, system and/or method according to the invention.

Other advantages, features and characteristics of the present invention, as well as methods of operation and functions of the related elements of the device, system, method, and computer readable medium and the combination of steps, parts and economies of manufacture, will become more apparent upon consideration of the following detailed description and the appended claims with reference to the accompanying drawings, the latter of which are briefly described hereinbelow.

### BRIEF DESCRIPTION OF THE DRAWINGS

The novel features which are believed to be characteristic of the device, system, method, and computer readable medium according to the present invention, as to the structure, organization, use, and method of operation, together with further objectives and advantages thereof, will be better understood from the following drawings in which presently preferred embodiments of the invention will now be illustrated by way of example. It is expressly understood, however, that the drawings are for the purpose of illustration and description only, and are not intended as a definition of the limits of the invention. In the accompanying drawings:
**Figure 1** is a schematic diagram of a universal lateral flow reader device according to a preferred embodiment of the invention;
**Figure 2** depicts three images of three different prior art cassettes made by different manufacturers, taken according to a preferred embodiment of the invention;
**Figure 3** depicts two different images of a single fluorescent lateral flow cassette: **(A)** is a first image taken with white LEDs on, color LEDs off, and an optical filter away from a camera; and **(B)** is a second image showing a test line, taken with the white LEDs off, the color LEDs on, and the optical filter in front of the camera; both taken according to a preferred embodiment of the invention;
**Figure 4** is an image of a universal lateral flow reader system, including a cellular telephone, according to another preferred embodiment of the invention;
**Figure 5** depicts two images of a fluorescent lateral flow cassette: **(A)** is a first image taken with a white LED on, and an ultraviolet (UV) LED off; and **(B)** is a second image taken with the white LED off, and the UV LED on; both taken with a long pass optical filter in front of the camera according to a further preferred embodiment of the invention; and
**Figure 6** depicts four flowcharts, each showing a workflow method for lighting and incubation: **(A)** and **(B)** are flowcharts with a cassette incubated outside of a reader device; and **(C)** and **(D)** are flowcharts with the cassette incubated inside of the reader device; all according to different preferred embodiments of the invention.

### DETAILED DESCRIPTION OF PREFERRED EMBODIMENTS

Preferred embodiments of the device, system, method, and computer readable medium according to the invention are alternately herein referred to, collectively and/or individually, as the universal lateral flow reader, device, system, method and/or computer readable medium (or simply as the reader, device system, method and/or computer readable medium). References to one or more of the reader, device, system, method and/or computer readable medium may, if and as appropriate, be understood by persons having ordinary skill in the art to apply, *mutatis mutandis,* to the others.

Persons skilled in the art will appreciate that although some of the components, relations, functionalities and applications of the reader, device, system, method and computer readable medium are not specifically referenced or described in conjunction with each other, they may be used or adapted for use in association therewith. The reader, device, system, method and computer readable medium described herein are suitable for use with each other, but they are not so limited.

### How It Works

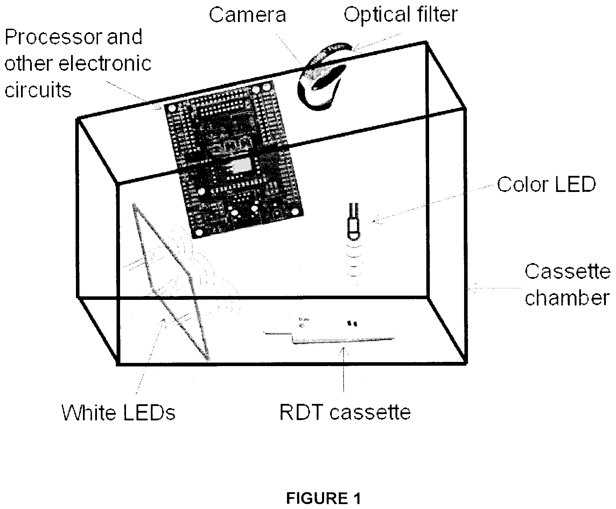

Figure 1 provides a schematic diagram of one reader device and/or system (and/or for use with a method) according to a preferred embodiment of the invention. It preferably includes a color charge-coupled device ("CCD") camera, white LEDs, color LEDs, an optical filter, a printed circuit board ("PCB"), and a lateral flow test cassette. The cassette is preferably enclosed in a chamber when a picture is taken. The white LEDs may have a wide illumination angle. Preferably, when on, the white LEDs may make the whole cassette chamber bright and provide a constant lighting environment for the camera. The color LEDs (and/or UV LEDs) are preferably used as an excitation source of one or more fluorophores in a fluorescence lateral flow test. The light of color LEDs may be collimated. The color LED light may illuminate only the membrane area of the cassette. The camera is preferably mounted high enough to take an image of the entire cassette. According to one preferred embodiment, the optical filter is an optical band pass filter which passes the fluorescent wavelength of the fluorophore.

Preferably, for gold lateral flow cassettes (and/or reflection signal tests), the white LEDs are on, the color LEDs are off, and the optical filter is switched away from the camera. The camera preferably takes an image of the entire cassette.

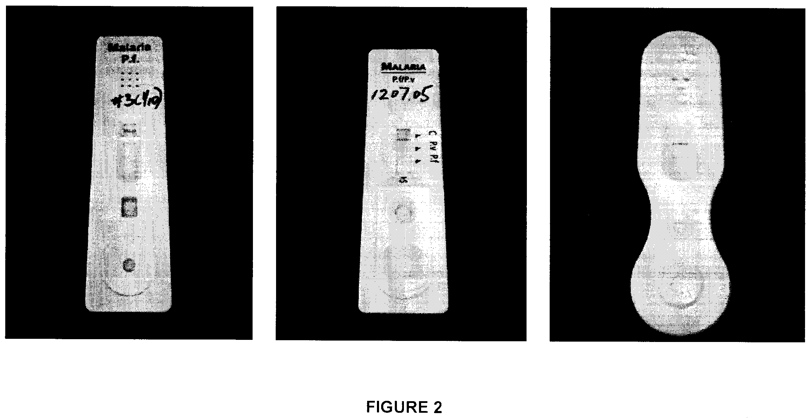

Figure 2 shows images of cassettes from three different manufacturers, as may be taken according to a preferred embodiment of the invention. Those bright color images, as preferably illuminated by the white LEDs, are analyzed by software in the microprocessor. Preferably, some of the functions of the image analysis software include: (1) cassette recognition which may be performed with reference to cassette features such as, for example, cassette size, aspect ratio, color, shape, letters, etc.; (2) membrane region identification for each type of cassette, i.e., in a preferred embodiment according to the invention, identifying a precise and/or approximate location of a membrane region for each cassette as (and cassette type which may be) inserted into the chamber; (3) measurement of the intensity of one or more test lines and/or control lines in the membrane region; and (4) determination of diagnostic results. Preferably, for each type of cassette, an assay cut-off value may be tested and/or predetermined, and pre-set as a reference value for and/or in the software. Preferably, in the aforesaid manner, the intensities of the control and test lines may be measured and determined, and the diagnostic results (such as positive, negative, invalid) can be determined and/or obtained.

Preferably, two or more images may be taken for each lateral flow cassette which is detected and/or determined to be of the fluorescent and/or emission signal type. The first image is preferably an image of the cassette with the white LED on, the color and/or UV LEDs off, and the optical filter away from the camera. This image is preferably used for automatic cassette recognition, which may preferably be based on information such as cassette size, shape, writings, and other specific features. Preferably, with the color and/or UV LEDs off, this image will not show any line in the membrane area for fluorescent lateral flow cassettes.

Preferably, the second image is taken with the white LEDs off, the color and/or UV LEDs on, and the optical filter in front of the camera. The color and/or UV LEDs preferably illuminate only the membrane area of the cassette, preferably to reduce an autofluorescence background signal such as from a plastic case of the cassette. Intensities of the fluorescent control line and/or test lines are preferably obtained in the second image.

The image processing software preferably combines the two images, and presents a user with the cassette type and/or the diagnostic results.

In doing so, the image processing software preferably accesses, references and/or consults one or more databases which are (a) local to the image processing software, to the device, to other components of the system, and/or to the computer readable medium, and/or (b) remote therefrom, with a communications element being used to remotely access, reference and/or consult them. The databases preferably take the form of one or more local, remote, distributed, congruent and/or peer-to-peer databases which are preferably be accessible by the image processing software and/or the device locally and/or over one or more of its regular wireless (and/or wired) communication networks, including terrestrial and/or satellite networks -- e.g., the Internet and cloud-based networks.

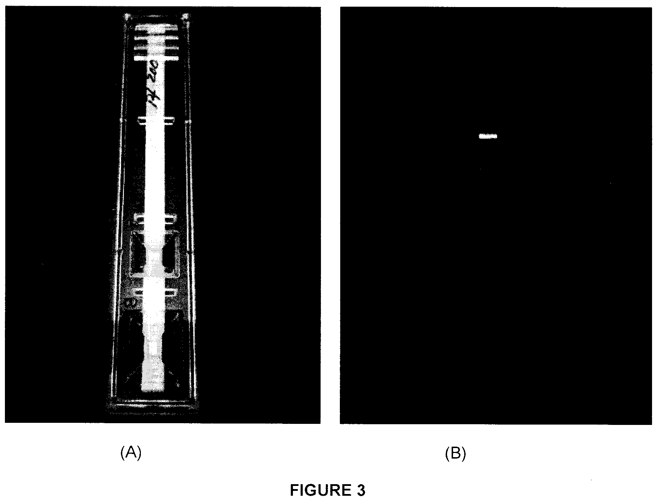

Figure 3 shows an example of two images such as may be taken of one type of fluorescent lateral flow cassette. Figure 3(A) shows an image of the cassette. Preferably, the Figure 3(A) image is taken with white LEDs on, color LEDs off, and optical filter away from the camera. The image in Figure 3(A) could preferably be used for cassette recognition. Figure 3(B) shows a fluorescent signal image of the same cassette. The Figure 3(B) image is preferably taken with white LEDs off, color LEDs on, and optical filter in front of the camera. Preferably, in the shown fluorescent lateral flow test, Europium dye is used. The image in Figure 3(B) could preferably be used for diagnostic test result analysis.

### Functional Check And Fluorescent Signal Calibration

A functional check and calibration ("FCC") cassette is preferably used to make sure the device, system and/or method are functional for both gold (reflection signal) and fluorescent (emission signal) lateral flow cassette test types. The FCC cassette preferably contains at least one color line and one fluorescent signal line. The plastic case of the FCC cassette may or may not be the same as that of a normal diagnostic cassette, as long as an associated software ("SW") algorithm [which may be provided according to one preferred embodiment of the invention] is preferably able to differentiate it therefrom. Preferably, the intensities of the color line and fluorescent line are pre-set, and should not appreciably change over a meaningful time relative to the expected lifetime(s) of the FCC cassette and/or the device according to the present invention. Preferably, to evaluate the device, the intensities of the two lines are tested. The criteria for functional check pass and/or failure is preferably pre-determined, and recorded in and/or accessed by the software.

### Other Preferred Embodiments

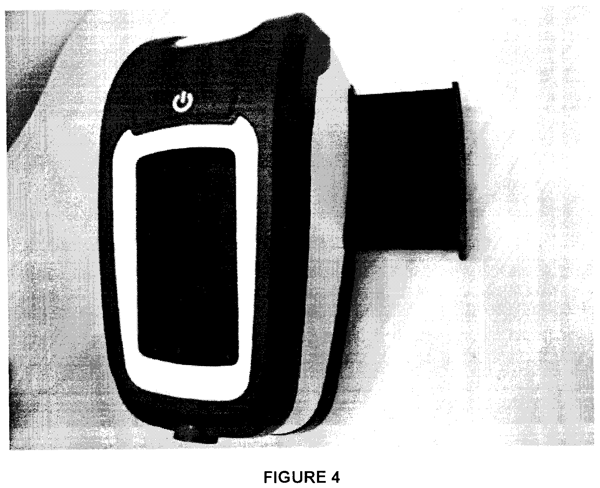

A lateral flow reader device, system and/or method according to one preferred embodiment of the invention may comprise, be used in conjunction with, and/or be based on a cell phone. Figure 4 shows this preferred embodiment. The cell phone's back camera may be used as the color camera according to this embodiment. Preferably, the cell phone's multiple functions (such as image taking, image processing, and/or information transferring) may afford benefits for the device, system and/or method according to this embodiment.

In this preferred embodiment, the cassette drawer and the enclosed cassette chamber preferably provide a constant lighting condition, with LEDs on, only when an image is being taken. Preferably, the device, system and/or method may be adapted such that the left channel audio signal from the cell phone may be used to control whether the white LEDs are on or off, and/or such that the right channel audio signal from the cell phone may be used to control whether the color and/or UV LEDs are on or off.

A device, system and/or method according to another preferred embodiment of the invention may be custom-designed and/or assembled by using separate parts and/or components, so as to afford the same, similar, or greater functionality than those comprising, used in conjunction with, and/or based on commercially available cell phones.

A further preferred embodiment of the device, system and/or method according to the invention may comprise, be used in conjunction with, and/or be based on a scanner device. In this embodiment, a scanning head may preferably be equipped with a red (R) LED, a green (G) LED, a blue (B) LED, and a UV LED. Preferably, for color images of gold (or reflection signal) lateral flow cassettes, only the RGB LEDs (or white LED) may be on when scanning. Preferably, for fluorescent (or emission signal) lateral flow cassette detection, two or more scans may be taken. Preferably, for cassette recognition, the first scan obtains a color image of the cassette, including its plastic case, with the RGB LEDs (or white LED) on and the UV LED off. The second scan preferably obtains the emitted fluorescent signal of the control line and the test line in the membrane area of the cassette, with the RGB LEDs (or white LED) off and the UV LED on. In this preferred embodiment, a band pass filter is placed in from of the image sensor when the UV LED is on.

Yet another preferred embodiment may be provided with a filter wheel which has two or more, and preferably several, optical band pass filters for different fluorophores and a through-hole (or clear) position for bright and/or white-light images. This embodiment may preferably also contain two or more, and preferably several, color and/or UV LEDs for different fluorophores. Whether each of these LEDs is on, or off, may preferably be controlled by the device software of this embodiment. Preferably, the filter wheel switch may be motorized, or the filter wheel may be turned manually. The filter wheel may preferably be turned automatically to the right filter depending on which LED(s) are on and/or which cassette is recognized.

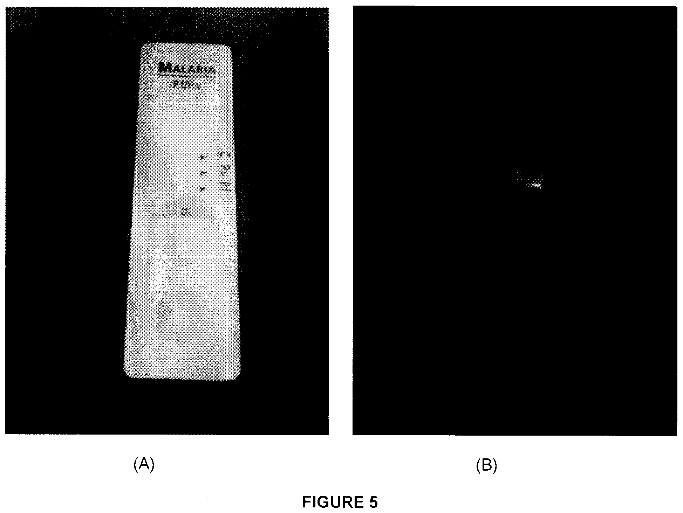

A yet further preferred embodiment of the invention may be provided without any optical filter sliding switch, without any optical filter wheel, and without any band pass filters. Instead, the device, system and/or method may comprise, be used in conjunction with, and/or be based on a white LED, a UV LED, and a long pass filter. Preferably, in this embodiment, the optical filter may be always placed in front of the camera. As an example, the wavelength of the UV LED may be about 375 nanometers (nm), and the cutting edge wavelength of the optical long pass filter may be about 420 nanometers (nm). In this embodiment of the invention, the fluorophore of the fluorescent lateral flow test preferably may be Europium dye. Figure 5 shows preferable images of a fluorescent lateral flow cassette according to this embodiment. Figure 5(A) shows a preferable image of the cassette when the white LED is on, the UV LED is off, and the 420 nanometer (nm) long pass filter is placed in front of camera. Figure 5(B) shows a preferable image of the cassette when the white LED is off, the UV LED is on, and the 420 nanometer (nm) long pass filter is placed in front of the camera. Preferably, the image in Figure 5(A) may be used for cassette recognition, and/or the image in Figure 5(B) may be used for fluorescent signal detection, according to this embodiment of the invention.

In still another preferred embodiment, the color camera may have a zoom-in function and/or be able to take a zoomed-in image of the membrane area when the color and/or UV LEDs are on, preferably for better fluorescence detection performance.

In a still further preferred embodiment, there may be provided one or more optical fibers and/or an optical fiber bundle to guide light to a specific area.

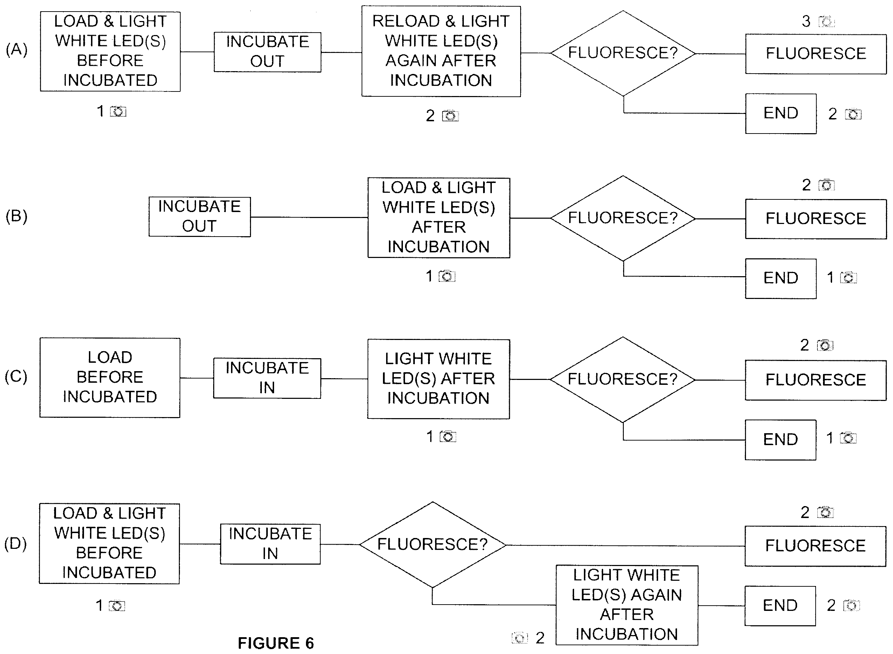

Figure 6 shows four potential workflows for use in or in association with the device, system and/or method according to the invention. Figures 6(A) and 6(B) show workflows where the cassette is preferably incubated outside of a reader device according to the invention. In Figure 6(A), the cassette is loaded into the device and the white LEDs are illuminated before the cassette is fully incubated. An image taken at this stage may be used for cassette recognition. In Figure 6(A), the cassette is removed from the device and reloaded again after incubation. In Figure 6(B), the cassette is first loaded into the device after incubation. In both cases, after incubation, the white LEDs are illuminated and images taken for cassette recognition.

Figures 6(C) and 6(D) show workflows where the cassette is preferably incubated inside of a reader device according to the invention. In both cases, the cassette is loaded into the device before it is fully incubated. Images taken when the white LEDs are first illuminated may be used for cassette recognition. In Figure 6(D) the white LEDs are first illuminated before the cassette is fully incubated, whereas in Figure 6(C) the white LEDs are first illuminated after incubation. In Figure 6(D), for reflection signal tests, the white LEDs are illuminated again after incubation.

In Figures 6(A) through 6(D), for reflection signal tests, images taken when the white LEDs are illuminated after incubation may be analyzed by the image processing software. For emission signal tests, the color and/or UV LEDs are illuminated to fluoresce the test and control lines and, with the optical filter in front of the camera, an image is taken for analysis by the image processing software.

In the workflow shown in Figure 6(A), two images are taken for reflection signal tests and three for emission signal tests. In the workflows shown in Figures 6(B) and 6(C), one image is taken for reflection signal tests and two for emission signal tests. In the workflow shown in Figure 6(D), two images are taken for reflection signal tests and two for emission signal tests.

### Computer Readable Medium

The computer readable medium (e.g., CD-ROM, DVD-ROM, flash USB stick, RAM, ROM, and/or other computer memory device) includes executable instructions which are physically stored thereon and which, upon execution, preferably encode processors to perform the method according to the invention.

All of the aforementioned, depicted and various structures, configurations, relationships, processes, utilities and the like may be, but are not necessarily, incorporated into and/or achieved by the invention. Any one or more of the aforementioned structures, configurations, relationships, processes, utilities and the like may be implemented in and/or by the invention, on their own, and/or without reference, regard or likewise implementation of any of the other aforementioned structures, configurations, relationships, processes, utilities and the like, in various permutations and combinations. The scope of the present invention is defined by the claims.

This concludes the description of presently preferred embodiments of the invention. The foregoing description has been presented for the purpose of illustration and is not intended to be exhaustive or to limit the invention to the precise form disclosed. Other modifications, variations and alterations are possible in light of the above teaching and will be apparent to those skilled in the art, and may be used in the design and manufacture of other embodiments according to the present invention , which is defined by the claims.

## Claims

1. A device for analysis of various types of cassettes for immunoassay rapid diagnostic tests, the device comprising:
one or more walls defining an enclosed chamber which is adapted to removably receive the various cassettes one at a time, each as a loaded cassette;
two or more light emitting diodes (LEDs) within the chamber, which comprise at least one reflection light emitting diode (LED) adapted to illuminate the loaded cassette, and at least one fluorescent LED adapted to fluoresce a membrane area of the loaded cassette;
an imaging element inside the chamber which automatically captures a first image of the loaded cassette when at least one of the LEDs is lit; and
at least one processor which, using the first image and a database that comprises at least one test protocol associated with each of the various test cassettes, automatically identifies an applicable one said test protocol for the loaded cassette;
wherein the processor automatically, after incubation of the loaded cassette and depending on the applicable one said test protocol:
(i) determines if the first image captures a post-incubation test signal from the loaded cartridge; and otherwise
(ii) lights the fluorescent LED to generate the test signal by emission, or the reflection LED to generate the test signal by reflection, from the loaded cassette and uses the imaging element to capture a second image of the loaded cassette and of the test signal; and wherein the processor automatically provides the test signal, in the first image or the second image, for analysis of the loaded cassette.

2. A device according to claim 1 , wherein the processor automatically analyzes the test signal with reference to the applicable one said test protocol for the loaded cassette; and
wherein preferably the test signal comprises a test line signal corresponding to a test line present on the loaded cassette after incubation, and the processor automatically measures an intensity of the test line signal.

3. A device according to claim 2, wherein the test signal comprises a control line signal corresponding to a control line present on the loaded cassette after incubation, and the processor automatically measures an intensity of the control line signal.

4. A device according to any one of claims 2 to 3, wherein the applicable one said test protocol comprises a predetermined assay threshold value, and wherein the processor analyzes the test signal with reference to the assay threshold value to automatically determine a diagnostic test result associated with the loaded cassette; and preferably further comprising an output device which automatically presents the diagnostic test result to a user of the device.

5. A device according to any one of claims 2 to 4, further comprising a memory onboard the device which stores one or more sets of executable instructions to encode the processor to automatically analyze the test signal as aforesaid.

6. A device according to any one of claims 1 to 4, further comprising a memory onboard the device which either: stores one or more sets of executable instructions to encode the processor to automatically identify the applicable one said test protocol as aforesaid; or
stores the database.

7. A device according to any one of claims 1 to 6, further comprising a communications element onboard the device which the processor automatically uses to remotely reference the database as aforesaid.

8. A device according to any one of claims 1 to 7, further comprising at least one optical filter, which is preferably an optical band pass filter, and wherein (i) the processor automatically ensures the optical filter is moved to an engaged position between the loaded cassette and the imaging element, before lighting a corresponding one said emission LED, and the imaging element then captures the test signal through the optical filter, and (ii) the processor automatically ensures the optical filter is moved to a disengaged position clear of the imaging element, before lighting the reflection LED, and the imaging element then captures the test signal clear of the optical filter; and
wherein preferably the optical filter is either mounted on a sliding switch which slides the optical filter between the engaged position and the disengaged position; or
mounted on a rotatable mechanism which rotates the optical filter between the engaged position and the disengaged position.

9. A device according to any one of claims 1 to 7, further comprising an optical long pass filter positioned between the loaded cassette and the imaging element, and wherein the imaging element captures the test signal through the optical long pass filter.

10. A device according to any one of claims 1 to 9, wherein said at least one reflection LED comprises at least one white LED; and/or
wherein said at least one fluorescent LED comprises at least one LED selected from an ultraviolet LED, a coloured LED, or a combination thereof.

11. A device according to any one of claims 1 to 10, wherein the processor identifies the applicable one said test protocol with reference to one or more of the following which are captured in the first image, and stored in the database, for the loaded cassette: one or more cassette dimensions, one or more cassette shapes, one or more detection line dimensions, one or more control line dimensions, one or more detection areas, one or more membrane areas, one or more control line positions, one or more test line positions, one or more cassette colors, one or more line colors, manufacturer indicia, product indicia, brand name indicia, application indicia, disease indicia, test type indicia, incubation time indicia, expected results indicia, barcodes, two-dimensional barcodes, labels, and other printed and written indicia; or preferably in addition or as an alternative the processor additionally identifies the applicable one said test protocol with reference to one or more of the following which are received from the loaded cassette, and stored in the database for the loaded cassette: magnetically stored data, fluorescence data, and radioactive signal data.

12. A device according to any one of claims 1 to 11, adapted for analysis of various lateral flow cassettes as the test cassettes.

13. A device according to any one of claims 1 to 12, adapted for use with a cellular telephone to provide at least one of said imaging element and said processor; and
wherein preferably left and right channel audio signals from the cellular telephone are adapted to turn the reflection LED and the fluorescent LED on and off.

14. A device according to any one of claims 1 to 12, wherein the imaging element comprises one or more scanning heads.

15. A system for analysis of various types of cassettes for immunoassay rapid diagnostic tests, the system comprising:
one or more walls defining an enclosed chamber which is adapted to selectively receive the various cassettes one at a time, each as a loaded cassette, in selectively removable relation;
two or more light emitting diodes (LEDs) within the chamber, which comprise at least one reflection light emitting diode (LED) adapted to illuminate the loaded cassette when lit, and at least one fluorescent LED adapted to fluoresce the loaded cassette when lit;
a database that comprises at least one test protocol associated with each of the various test cassettes;
a cellular telephone received by the walls of the chamber, with the cellular telephone comprising: a camera inside the chamber which automatically captures a first image of the loaded cassette when at least one of the LEDs is lit; and at least one processor which, with reference to the first image and in communication with the database, automatically identifies an applicable one said test protocol for the loaded cassette;
wherein the processor automatically, after incubation of the loaded cassette and depending on the applicable one said test protocol:
(i) determines if the first image captures a post-incubation test signal from the loaded cartridge; and otherwise
(ii) lights the fluorescent LED to generate the test signal by emission, or the reflection LED to generate the test signal by reflection, from the loaded cassette and uses the imaging element to capture a second image of the loaded cassette and of the test signal; and wherein the processor automatically provides the test signal, in the first image or the second image, for analysis of the loaded cassette; and
wherein preferably left and right channel audio signals from the cellular telephone are adapted to turn the reflection LED and the fluorescent LED on and off.

16. A method for analysis of various types of cassettes for immunoassay rapid diagnostic tests, the method comprising:
a receiving step of removably receiving the various cassettes one at a time, each as a loaded cassette within an enclosed chamber which is defined by one or more walls;
a light emitting diode (LED) step of providing two or more LEDs within the chamber, which comprise at least one reflection LED adapted to illuminate the loaded cassette, and at least one fluorescent LED adapted to fluoresce a membrane area of the loaded cassette;
an imaging step of automatically capturing a first image of the loaded cassette, when at least one of the LEDs is lit, using an imaging element inside the chamber;
a database step of providing a database which comprises at least one test protocol associated with each of the various test cassettes; and
a processing step wherein at least one processor uses the first image and the database to automatically identify an applicable one said test protocol for the loaded cassette;
wherein in the processing step, the processor automatically, after incubation of the loaded cassette and depending on the applicable one said test protocol:
(i) determines if the first image captures a post-incubation test signal from the loaded cartridge; and otherwise
(ii) lights the fluorescent LED to generate the test signal by emission, or the reflection LED to generate the test signal by reflection, from the loaded cassette and uses the imaging element to capture a second image of the loaded cassette and of the test signal; and wherein in the processing step, the processor automatically provides the test signal, in the first image or the second image, for analysis of the loaded cassette; and preferably
wherein, in the processing step, the processor automatically analyzes the test signal with reference to the applicable one said test protocol for the loaded cassette; and yet more preferably
the test signal comprises a test line signal corresponding to a test line present on the loaded cassette after incubation, and the processor automatically measures an intensity of the test line signal.

17. A method according claim 16 wherein, in the processing step, the test signal comprises a control line signal corresponding to a control line present on the loaded cassette after incubation, and the processor automatically measures an intensity of the control line signal; and preferably in addition or as an alternative, in the processing step, the applicable one said test protocol comprises a predetermined assay threshold value, and the processor analyzes the test signal with reference to the assay threshold value to automatically determine a diagnostic test result associated with the loaded cassette; and yet more preferably
the method further comprising a presentation step, wherein the diagnostic test result is automatically presented using an output device.

18. A method according to any one of claims 16 to 17 wherein, before the processing step, one or more sets of executable instructions are stored in a memory and, in the processing step, the executable instructions encode the processor to automatically analyze the test signal as aforesaid; or alternatively
wherein, before the processing step, one or more sets of executable instructions are stored in a memory and, in the processing step, the executable instructions encode the processor to automatically identify the applicable one said test protocol as aforesaid.

19. A method according to any one of claims 16 to 18 wherein, in the processing step, the processor automatically uses a communications element to remotely reference the database as aforesaid.

20. A method according to any one of claims 16 to 19, further comprising either:
a filtering step of providing at least one optical filter, and wherein in the processing step, (i) the processor automatically ensures the optical filter is moved to an engaged position between the loaded cassette and the imaging element, before lighting a corresponding one said emission LED, and the imaging element then captures the test signal through the optical filter, and (ii) the processor automatically ensures the optical filter is moved to a disengaged position clear of the imaging element, before lighting the reflection LED, and the imaging element then captures the test signal clear of the optical filter; or
a filtering step of providing an optical long pass filter positioned between the loaded cassette and the imaging element, and wherein in the processing step, the imaging element captures the test signal through the optical long pass filter.

21. A method according to any one of claims 16 to 20 wherein, in the LED step, at least one white LED is provided as said at least one reflection LED; and/or
at least one fluorescent LED selected from an ultraviolet LED, a coloured LED, or a combination thereof is provided.

22. A method according to any one of claims 16 to 21 wherein, in the processing step, the processor identifies the applicable one said test protocol with reference to one or more of the following which are captured in the first image, and stored in the database, for the loaded cassette: one or more cassette dimensions, one or more cassette shapes, one or more detection line dimensions, one or more control line dimensions, one or more detection areas, one or more membrane areas, one or more control line positions, one or more test line positions, one or more cassette colors, one or more line colors, manufacturer indicia, product indicia, brand name indicia, application indicia, disease indicia, test type indicia, incubation time indicia, expected results indicia, barcodes, two-dimensional barcodes, labels, and other printed and written indicia; and preferably in addition or as an alternative, in the processing step, the processor additionally identifies the applicable one said test protocol with reference to one or more of the following which are received from the loaded cassette, and stored in the database for the loaded cassette: magnetically stored data, fluorescence data, and radioactive signal data.

23. A method according to any one of claims 16 to 22 wherein either: in at least one of the imaging step and the processing step, a cellular telephone is provided as said imaging element and/or as said processor; and preferably
left and right channel audio signals from the cellular telephone turn the reflection LED and the fluorescent LED on and off; or
in the imaging step, one or more scanning heads are provided as at least part of said imaging element.

24. A computer readable medium for analysis of various types of cassettes for immunoassay rapid diagnostic tests, configured to be used with the device of claim 1; with the computer readable medium comprising executable instructions which are physically stored thereon and which, upon execution, encode at least one processor to automatically:
capture a first image of the loaded cassette using the imaging element when at least one of the LEDs is lit;
identify, using the first image and the database, an applicable one said test protocol for the loaded cassette;
after incubation of the loaded cassette and depending on the applicable one said test protocol:
(i) determine if the first image captures a post-incubation test signal from the loaded cartridge; and otherwise (ii) light the fluorescent LED to generate the test signal by emission, or the reflection LED to generate the test signal by reflection, from the loaded cassette and uses the imaging element to capture a second image of the loaded cassette and of the test signal; and
provide the test signal, in the first image or the second image, for analysis of the loaded cassette.

## Patentansprüche

1. Vorrichtung zur Analyse verschiedener Arten von Kassetten für schnelle diagnostische Immunoassay-Tests, wobei die Vorrichtung umfasst:
Eine oder mehrere Wände, die eine umschlossene Kammer definieren, die angepasst ist, die verschiedenen Kassetten eine nach der anderen entfernbar, jede als eine geladene Kassette, aufzunehmen;
zwei oder mehrere Leuchtdioden (LEDs) innerhalb der Kammer, welche zumindest eine Reflexions-Leuchtdiode (LED) umfassen, die angepasst ist, die geladene Kassette zu beleuchten, und zumindest eine fluoreszierende LED, die angepasst ist, eine Membranfläche der geladenen Kassette zu fluoreszieren;
ein bildgebendes Element innenseitig der Kammer, das ein erstes Bild der geladenen Kassette automatisch erfasst, wenn zumindest eins der LEDs leuchtet; und
zumindest einen Prozessor, der, unter Verwendung des ersten Bildes und einer Datenbank, die zumindest ein Testprotokoll umfasst, das mit jeder der verschiedenen Testkassetten assoziiert ist, automatisch ein anwendbares Protokoll des Testprotokolls für die geladene Kassette identifiziert;
wobei der Prozessor automatisch, nach Inkubation der geladenen Kassette und abhängig vom anwendbaren Protokoll des Testprotokolls:
(i) ermittelt, ob das erste Bild ein Post-Inkubations-Testsignal von der geladenen Kassette erfasst; und andernfalls
(ii) die fluoreszierende LED, um das Testsignal durch Emission zu generieren, oder die Reflexions-LED anmacht, um das Testsignal durch Reflexion von der geladenen Kassette zu generieren und das bildgebende Element verwendet, um ein zweites Bild der geladenen Kassette und vom Testsignal zu erfassen; und wobei der Prozessor das Testsignal, im ersten Bild oder im zweiten Bilde, zur Analyse der geladenen Kassette automatisch bereitstellt.

2. Vorrichtung nach Anspruch 1, wobei der Prozessor das Testsignal mit Bezug auf das anwendbare Protokoll des Testprotokolls für die geladene Kassette automatisch analysiert; und
wobei das Testsignal vorzugsweise ein Testliniensignal umfasst, das einer Testlinie entspricht, die an der geladenen Kassette nach Inkubation zugegen ist, und der Prozessor eine Intensität des Testliniensignals automatisch misst.

3. Vorrichtung nach Anspruch 2, wobei das Testsignal ein Kontrollliniensignal umfasst, das einer Kontrolllinie entspricht, die nach Inkubation an der geladenen Kassette zugegen ist, und der Prozessor eine Intensität des Kontrollliniensignals automatisch misst.

4. Vorrichtung nach einem der Ansprüche 2 bis 3, wobei das anwendbare Protokoll des Testprotokolls einen vorbestimmten Assay-Schwellenwert umfasst, und wobei der Prozessor das Testsignal mit Bezug auf den Assay-Schwellenwert analysiert, um automatisch ein diagnostisches Testergebnis zu bestimmen, das mit der geladenen Kassette assoziiert ist; und vorzugsweise ferner eine Ausgabevorrichtung umfasst, welche das diagnostische Testergebnis einem Benutzer der Vorrichtung automatisch präsentiert.

5. Vorrichtung nach einem der Ansprüche 2 bis 4, die ferner einen Speicher an Bord der Vorrichtung umfasst, der einen oder mehrere Sätze ausführbarer Befehle speichert, um den Prozessor zu codieren, das Testsignal wie vorstehend automatisch zu analysieren.

6. Vorrichtung nach einem der Ansprüche 1 bis 4, das ferner einen Speicher an Bord der Vorrichtung umfasst, der entweder: einen oder mehrere Sätze von ausführbaren Befehlen speichert, um den Prozessor zu codieren, das anwendbare Protokoll des Testprotokolls wie vorstehend automatisch zu identifizieren; oder die Datenbank speichert.

7. Vorrichtung nach einem der Ansprüche 1 bis 6, die ferner ein Kommunikationselement an Bord der Vorrichtung umfasst, das der Prozessor automatisch verwendet, um die Datenbank wie vorstehend aus der Ferne zu referenzieren.

8. Vorrichtung nach einem der Ansprüche 1 bis 7, die ferner zumindest einen optischen Filter umfasst, der vorzugsweise ein optischer Bandpaßfilter ist, und wobei (i) der Prozessor automatisch sicherstellt, dass der optische Filter in eine eingerückte Position zwischen der geladenen Kassette und dem bildgebenden Element bewegt wird, bevor eine entsprechende LED der Emissions-LED angemacht wird, und das bildgebende Element erfasst dann das Testsignal durch den optischen Filter, und (ii) der Prozessor automatisch sicherstellt, dass der optische Filter in eine ausgerückte Position weg vom bildgebenden Element bewegt wird, bevor die Reflexions-LED angemacht wird, und das bildgebende Element erfasst dann das Testsignal weg vom optischen Filter; und
wobei der optische Filter vorzugsweise auf einem Schiebeschalter montiert ist, der den optischen Filter zwischen der eingerückten Position und der ausgerückten Position verschiebt; oder
auf einem drehbaren Mechanismus montiert ist, der den optischen Filter zwischen der eingerückten Position und der ausgerückten Position dreht.

9. Vorrichtung nach einem der Ansprüche 1 bis 7, die ferner einen optischen Langpassfilter umfasst, der zwischen der geladenen Kassette und dem bildgebenden Element positioniert ist, und wobei das bildgebende Element das Testsignal durch den optischen Langpassfilter erfasst.

10. Vorrichtung nach einem der Ansprüche 1 bis 9, wobei die zumindest eine Reflexions-LED zumindest eine weiße LED umfasst; und/oder
wobei die zumindest eine fluoreszierende LED zumindest eine LED umfasst, die aus einer ultravioletten LED, einer farbigen LED oder einer Kombination davon selektiert ist.

11. Vorrichtung nach einem der Ansprüche 1 bis 10, wobei der Prozessor das anwendbare Protokoll des Testprotokolls mit Bezugnahme auf eins oder mehrere der Folgenden, die im ersten Bild erfasst sind, und in der Datenbank gespeichert sind, für die geladene Kassette identifiziert: Eine oder mehrere Kassetten-Abmessungen, eine oder mehrere Kassetten-Formen, eine oder mehrere Erkennungslinien-Abmessungen, eine oder mehrere Kontrolllinien-Abmessungen, eine oder mehrere Erkennungsflächen, eine oder mehrere Membranflächen, eine oder mehrere Kontrolllinien-Positionen, eine oder mehrere Testlinien-Positionen, eine oder mehrere Kassettenfarben, eine oder mehrere Linienfarben, Herstellerzeichen, Produktzeichen, Markennamen-Zeichen, Anwendungszeichen, Krankheitszeichen, Testart-Zeichen, Inkubationszeit-Zeichen, Zeichen für erwartete Ergebnisse, Barcodes, zweidimensionale Barcodes, Etiketten, und andre gedruckte und geschriebene Zeichen; oder vorzugsweise zusätzlich oder als eine Alternative identifiziert der Prozessor zusätzlich das anwendbare Protokoll des Testprotokolls mit Bezugnahme auf eins oder mehrere der Folgenden, die ab der geladenen Kassette empfangen und in der Datenbank für die geladene Kassette gespeichert wurden: magnetisch gespeicherte Daten, fluoreszierende Daten und radioaktive Signaldaten.

12. Vorrichtung nach einem der Ansprüche 1 bis 11, die für Analyse verschiedener "Lateral Flow" Kassetten als die Testkassetten angepasst ist.

13. Vorrichtung nach einem der Ansprüche 1 bis 12, die zur Verwendung mit einem zellularen Telefon angepasst ist, um zumindest eines des bildgebenden Elements und des Prozessors bereitzustellen; und
wobei vorzugsweise linke und rechte Kanal-Audiosignale vom zellularen Telefon angepasst sind, die Reflexions-LED und die fluoreszierende LED ein- und auszuschalten.

14. Vorrichtung nach einem der Ansprüche 1 bis 12, wobei das bildgebende Element einen oder mehrere Abtastköpfe umfasst.

15. System zur Analyse verschiedener Arten von Kassetten für schnelle diagnostische Immunoassay-Tests, wobei das System umfasst:
Eine oder mehrere Wände, die eine umschlossene Kammer definieren, die angepasst ist, die verschiedenen Kassetten eine nach der anderen, jede als eine geladene Kassette, in selektiv entfernbarer Beziehung, selektiv aufzunehmen;
zwei oder mehrere Leuchtdioden (LEDs) innerhalb der Kammer, welche zumindest eine Reflexions-Leuchtdiode (LED), die angepasst ist, die geladene Kassette, wenn angemacht, zu beleuchten, und zumindest eine fluoreszierende LED umfassen, die angepasst ist, die geladenen Kassette, wenn angemacht, zu fluoreszieren;
eine Datenbank, die zumindest ein Testprotokoll umfasst, das mit jeder der verschiedenen Testkassetten assoziiert ist;
ein zellulares Telefon, das von den Wänden der Kammer aufgenommen wird, wobei das zellulare Telefon umfasst: Eine Kamera innenseitig der Kammer, das automatisch ein erstes Bild der geladenen Kassette erfasst, wenn zumindest eine der LEDs angemacht ist; und zumindest einen Prozessor, der, mit Bezug auf das erste Bild und in Kommunikation mit der Datenbank, automatisch ein anwendbares Protokoll des Testprotokolls für die geladene Kassette identifiziert;
wobei der Prozessor automatisch, nach Inkubation der geladenen Kassette und abhängig vom anwendbaren Protokoll des Testprotokolls:
(i) ermittelt, ob das erste Bild ein Post-Inkubations-Testsignal von der geladenen Kassette erfasst; und andernfalls
(ii) die fluoreszierende LED, um das Testsignal durch Emission zu generieren, oder die Reflexions-LED anmacht, um das Testsignal durch Reflexion von der geladenen Kassette zu generieren und das bildgebende Element verwendet, um ein zweites Bild der geladenen Kassette und vom Testsignal zu erfassen; und wobei der Prozessor das Testsignal, im ersten Bild oder im zweiten Bild, zur Analyse der geladenen Kassette automatisch bereitstellt; und
wobei vorzugsweise linke und rechte Kanal-Audiosignale vom zellularen Telefon angepasst sind, die Reflexions-LED und die fluoreszierende LED ein- und auszuschalten.

16. Verfahren zur Analyse verschiedener Arten von Kassetten für schnelle diagnostische Immunoassay-Tests, wobei das Verfahren umfasst:
Einen Aufnahmeschritt zum entfernbaren Aufnehmen der verschiedenen Kassetten eine nach der anderen, jede als eine geladene Kassette innerhalb einer umschlossenen Kammer, die durch eine oder mehrere Wände definiert ist;
Einen Leuchtdioden-Schritt (LED-) der Bereitstellung von zwei oder mehreren LEDs innerhalb der Kammer, welche zumindest eine Reflexions-LED, die angepasst ist, die geladene Kassette zu beleuchten und zumindest eine fluoreszierende LED umfassen, die angepasst ist, eine Membranfläche der geladenen Kassette zu fluoreszieren;
einen bildgebenden Schritt der automatischen Erfassung eines ersten Bildes der geladenen Kassette, wenn zumindest eine der LEDs leuchtet, unter Verwendung eines bildgebenden Elements innenseitig der Kammer;
einen Datenbank-Schritt der Bereitstellung einer Datenbank, die zumindest ein Testprotokoll umfasst, das mit jeder der verschiedenen Testkassetten assoziiert ist; und
einen Verarbeitungsschritt, wobei zumindest ein Prozessor das erste Bild und die Datenbank verwendet, um automatisch ein anwendbares Protokoll des Testprotokolls für die geladenen Kassette zu identifizieren;
wobei beim Verarbeitungsschritt der Prozessor automatisch, nach Inkubation der geladenen Kassette und abhängig vom anwendbaren Protokoll des Testprotokolls:
(i) ermittelt, ob das erste Bild ein Post-Inkubations-Testsignal von der geladenen Kassette erfasst; und andernfalls
(ii) die fluoreszierende LED, um das Testsignal durch Emission zu generieren, oder die Reflexions-LED anmacht, um das Testsignal durch Reflexion von der geladenen Kassette zu generieren und das bildgebende Element verwendet, um ein zweites Bild der geladenen Kassette und vom Testsignal zu erfassen; und wobei beim Verarbeitungsschritt der Prozessor das Testsignal, im ersten Bild oder im zweiten Bild, zur Analyse der geladenen Kassette automatisch bereitstellt; und vorzugsweise
wobei, beim Verarbeitungsschritt, der Prozessor das Testsignal mit Bezug auf das anwendbare Protokoll für die geladene Kassette automatisch analysiert; und noch bevorzugter
das Testsignal ein Testliniensignal umfasst, das einer Testlinie entspricht, die an der geladenen Kassette nach Inkubation zugegen ist, und der Prozessor eine Intensität des Testliniensignals automatisch misst.

17. Verfahren nach Anspruch 16, wobei, beim Verarbeitungsschritt, das Testsignal ein Kontrollliniensignal umfasst, das einer Kontrolllinie entspricht, die an der geladenen Kassette nach Inkubation zugegen ist, und der Prozessor automatisch eine Intensität des Kontrollliniensignals misst; und vorzugsweise zusätzlich oder als eine Alternative, beim Verarbeitungsschritt, das anwendbare Protokoll des Testprotokolls einen vorbestimmten Assay-Schwellenwert umfasst, und der Prozessor das Testsignal mit Bezug auf den Assay-Schwellenwert analysiert, um automatisch ein diagnostisches Testergebnis zu bestimmen, das mit der geladenen Kassette assoziiert ist; und noch bevorzugter
das Verfahren ferner einen Darstellungsschritt umfasst, wobei das diagnostische Testergebnis unter Verwendung einer Ausgabevorrichtung automatisch dargestellt wird.

18. Verfahren nach irgendeinem der Ansprüche 16 bis 17 wobei, vor dem Verarbeitungsschritt, ein oder mehrere Sätze von ausführbaren Befehlen in einem Speicher gespeichert sind und, beim Verarbeitungsschritt, die ausführbaren Befehle den Prozessor codieren, um das Testsignal automatisch wie vorstehend zu analysieren; oder alternativ
wobei, vor dem Verarbeitungsschritt, ein oder mehrere Sätze von ausführbaren Befehlen in einem Speicher gespeichert sind und, beim Verarbeitungsschritt, die ausführbaren Befehle den Prozessor codieren, um das anwendbare Protokoll des Testprotokolls wie vorstehend automatisch zu identifizieren.

19. Verfahren nach irgendeinem der Ansprüche 16 bis 18, wobei, beim Verarbeitungsschritt, der Prozessor automatisch ein Kommunikationselement verwendet, um die Datenbank wie vorstehend aus der Ferne zu referenzieren.

20. Verfahren nach irgendeinem der Ansprüche 16 bis 19, ferner umfassend, entweder:
Einen filternden Schritt der Bereitstellung zumindest eines optischen Filters, und wobei beim Verarbeitungsschritt, (i) der Prozessor automatisch sicherstellt, dass der optische Filter in eine eingerückte Position zwischen der geladenen Kassette und dem bildgebenden Element bewegt wird, bevor eine entsprechende LED der Emissions-LED angemacht wird, und das bildgebende Element erfasst dann das Testsignal durch den optischen Filter, und (ii) der Prozessor automatisch sicherstellt, dass der optische Filter in eine ausgerückte Position weg vom bildgebenden Element bewegt wird, bevor die Reflexions-LED angemacht wird, und das bildgebende Element erfasst dann das Testsignal weg vom optischen Filter; oder
einen filternden Schritt der Bereitstellung eines optischen Langpassfilters, der zwischen der geladenen Kassette und dem bildgebenden Element positioniert ist, und wobei beim Verarbeitungsschritt das bildgebende Element das Testsignal durch den optischen Langpassfilter erfasst.

21. Verfahren nach irgendeinem der Ansprüche 16 bis 20, wobei, beim LED-Schritt, zumindest eine weiße LED als das zumindest eine Reflexion-LED bereitgestellt ist; und/oder
zumindest eine fluoreszierende, die aus einer ultravioletten LED, einer farbigen LED oder einer Kombination davon selektiert ist, bereitgestellt ist.

22. Verfahren nach irgendeinem der Ansprüche 16 bis 21, wobei, beim Verarbeitungsschritt, der Prozessor das anwendbare Protokoll des Testprotokolls mit Bezugnahme auf eins oder mehrere der Folgenden, die im ersten Bild erfasst sind, und in der Datenbank gespeichert sind, für die geladene Kassette identifiziert: Eine oder mehrere Kassetten-Abmessungen, eine oder mehrere Kassetten-Formen, eine oder mehrere Erkennungslinien-Abmessungen, eine oder mehrere Kontrolllinien-Abmessungen, eine oder mehrere Erkennungsflächen, eine oder mehrere Membranflächen, eine oder mehrere Kontrolllinien-Positionen, eine oder mehrere Testlinien-Positionen, eine oder mehrere Kassettenfarben, eine oder mehrere Linienfarben, Herstellerzeichen, Produktzeichen, Markennamen-Zeichen, Anwendungszeichen, Krankheitszeichen, Testart-Zeichen, Inkubationszeit-Zeichen, Zeichen für erwartete Ergebnisse, Barcodes, zweidimensionale Barcodes, Etiketten, und andre gedruckte und geschriebene Zeichen; und vorzugsweise zusätzlich oder als eine Alternative,
beim Verarbeitungsschritt, identifiziert der Prozessor zusätzlich das anwendbare Protokoll des Testprotokolls mit Bezugnahme auf eins oder mehrere der Folgenden, die ab der geladenen Kassette empfangen und in der Datenbank für die geladene Kassette gespeichert wurden: magnetisch gespeicherte Daten, fluoreszierende Daten und radioaktive Signaldaten.

23. Verfahren nach irgendeinem der Ansprüche 16 bis 22, wobei entweder: Beim zumindest einen des bildgebenden Schritts und des Verarbeitungsschritts, ein zellulares Telefon als das bildgebende Element und/oder als der Prozessor bereitgestellt wird; und vorzugsweise
linke und rechte Kanal-Audiosignale vom zellularen Telefon angepasst sind, die Reflexions-LED und die fluoreszierende LED ein- und auszuschalten; oder
beim bildgebenden Schritt sind ein oder mehrere Abtastköpfe als zumindest Teil des bildgebenden Elements bereitgestellt.

24. Computerlesbares Medium zur Analyse verschiedener Arten von Kassetten für schnelle diagnostische Immunoassay-Tests, konfiguriert mit der Vorrichtung nach Anspruch 1 verwendet zu werden;
wobei computerlesbare Medium ausführbare Befehle umfasst, die physikalisch darauf gespeichert sind und die, nach Ausführung zumindest einen Prozessor codieren, um automatisch:
Ein erstes Bild der geladenen Kassette unter Verwendung des bildgebenden Elements zu erfassen, wenn zumindest eins der LEDs angemacht ist;
Identifizieren, unter Verwendung des ersten Bildes und der Datenbank, eines anwendbaren Protokolls des Testprotokolls für die geladene Kassette;
nach Inkubation der geladenen Kassette und abhängig vom anwendbaren Protokoll des Testprotokolls:
(i) Ermitteln, ob das erste Bild ein Post-Inkubation-Testsignal von der geladenen Kassette erfasst; und andernfalls (ii) die fluoreszierende LED, um das Testsignal durch Emission zu generieren, oder die Reflexions-LED anmacht, um das Testsignal durch Reflexion, ab der geladenen Kassette, zu generieren und das bildgebende Element verwendet, um ein zweites Bild der geladenen Kassette und des Testsignals zu erfassen; und
Bereitstellen des Testsignals, im ersten Bild oder im zweiten Bild, zur Analyse der geladenen Kassette.

## Revendications

1. Dispositif pour l'analyse de divers types de cassettes pour des tests de diagnostic rapide par immunoessais, le dispositif comprenant :
une ou plusieurs parois définissant une chambre fermée qui est conçue pour recevoir de manière amovible les différentes cassettes une à la fois, chacune sous forme de cassette chargée ;
deux ou plusieurs diodes électroluminescentes (DEL) à l'intérieur de la chambre, qui comprennent au moins une diode électroluminescente (DEL) à réflexion conçue pour éclairer la cassette chargée, et au moins une DEL fluorescente conçue pour faire fluorescer une zone de membrane de la cassette chargée ;
un élément de formation d'image à l'intérieur de la chambre qui capte automatiquement une première image de la cassette chargée lorsqu'au moins l'une des DEL est allumée ; et
au moins un processeur qui, en utilisant la première image et une base de données qui comprend au moins un protocole de test associé à chacune des différentes cassettes de test, identifie automatiquement un dit protocole de test applicable pour la cassette chargée ;
dans lequel le processeur automatiquement, après incubation de la cassette chargée et en fonction dudit protocole de test applicable :
(i) détermine si la première image capte un signal de test post-incubation provenant de la cassette chargée ; et dans le cas contraire
(ii) allume la DEL fluorescente pour générer le signal de test par émission, ou la DEL de réflexion pour générer le signal de test par réflexion, à partir de la cassette chargée et utilise l'élément de formation d'image pour capter une deuxième image de la cassette chargée et du signal de test ; et dans lequel le processeur fournit automatiquement le signal de test, dans la première image ou la deuxième image, pour l'analyse de la cassette chargée.

2. Dispositif selon la revendication 1, dans lequel le processeur analyse automatiquement le signal de test en référence audit protocole de test applicable pour la cassette chargée ; et
dans lequel de préférence le signal de test comprend un signal de ligne de test correspondant à une ligne de test présente sur la cassette chargée après incubation, et le processeur mesure automatiquement une intensité du signal de ligne de test.

3. Dispositif selon la revendication 2, dans lequel le signal de test comprend un signal de ligne de contrôle correspondant à une ligne de contrôle présente sur la cassette chargée après incubation, et le processeur mesure automatiquement une intensité du signal de ligne de contrôle.

4. Dispositif selon l'une quelconque des revendications 2 à 3, dans lequel ledit protocole de test applicable comprend une valeur de seuil d'essai prédéterminée, et dans lequel le processeur analyse le signal de test en référence à la valeur de seuil d'essai pour déterminer automatiquement un résultat de test de diagnostic associé à la cassette chargée ; et comprenant de préférence en outre un dispositif de sortie qui présente automatiquement le résultat de test de diagnostic à un utilisateur du dispositif.

5. Dispositif selon l'une quelconque des revendications 2 à 4, comprenant en outre une mémoire à bord du dispositif qui stocke un ou plusieurs ensembles d'instructions exécutables pour coder le processeur afin d'analyser automatiquement le signal de test comme indiqué ci-dessus.

6. Dispositif selon l'une quelconque des revendications 1 à 4, comprenant en outre une mémoire à bord du dispositif qui soit : stocke un ou plusieurs ensembles d'instructions exécutables pour coder le processeur afin d'identifier automatiquement ledit protocole de test applicable comme mentionné ci-dessus ; soit stocke la base de données.

7. Dispositif selon l'une quelconque des revendications 1 à 6, comprenant en outre un élément de communication à bord du dispositif que le processeur utilise automatiquement pour référencer à distance la base de données comme indiqué ci-dessus.

8. Dispositif selon l'une quelconque des revendications 1 à 7, comprenant en outre au moins un filtre optique, qui est de préférence un filtre passe-bande optique, et dans lequel (i) le processeur vérifie automatiquement que le filtre optique est déplacé vers une position engagée entre la cassette chargée et l'élément de formation d'image, avant d'allumer une dite DEL d'émission correspondante, et l'élément de formation d'image capte alors le signal de test à travers le filtre optique, et (ii) le processeur vérifie automatiquement que le filtre optique est déplacé vers une position désengagée à l'écart de l'élément de formation d'image, avant d'allumer la DEL de réflexion, et l'élément de formation d'image capte alors le signal de test à l'écart du filtre optique ; et
dans lequel, de préférence, le filtre optique est soit monté sur un commutateur coulissant qui fait glisser le filtre optique entre la position engagée et la position désengagée ;
soit monté sur un mécanisme rotatif qui fait tourner le filtre optique entre la position engagée et la position désengagée.

9. Dispositif selon l'une quelconque des revendications 1 à 7, comprenant en outre un filtre optique passe-long positionné entre la cassette chargée et l'élément de formation d'image, et dans lequel l'élément de formation d'image capte le signal de test à travers le filtre optique passe-haut.

10. Dispositif selon l'une quelconque des revendications 1 à 9, dans lequel ladite au moins une DEL de réflexion comprend au moins une DEL blanche ; et/ou
dans lequel ladite au moins une DEL fluorescente comprend au moins une DEL sélectionnée parmi une DEL ultraviolette, une DEL colorée ou une combinaison de celles-ci.

11. Dispositif selon l'une quelconque des revendications 1 à 10, dans lequel le processeur identifie ledit protocole de test applicable en référence à un ou plusieurs des éléments suivants qui sont saisis dans la première image, et stockés dans la base de données, pour la cassette chargée : une ou plusieurs dimensions de cassette, une ou plusieurs formes de cassette, une ou plusieurs dimensions de ligne de détection, une ou plusieurs dimensions de ligne de contrôle, une ou plusieurs zones de détection, une ou plusieurs zones de membrane, une ou plusieurs positions de ligne de contrôle, une ou plusieurs positions de ligne de test, une ou plusieurs couleurs de cassette, une ou plusieurs couleurs de ligne, des indications de fabricant, des indications de produit, des indications de marque, des indications d'application, des indications de maladie, des indications de type de test, des indications de temps d'incubation, des indications de résultats attendus, des codes à barres, des codes à barres bidimensionnels, des étiquettes et d'autres indications imprimées et écrites ; ou de préférence en plus ou en alternative, le processeur identifie en outre ledit protocole de test applicable en référence à un ou plusieurs des éléments suivants qui sont reçus de la cassette chargée, et stockés dans la base de données pour la cassette chargée : données stockées magnétiquement, données de fluorescence, et données de signal radioactif.

12. Dispositif selon l'une quelconque des revendications 1 à 11, conçu pour l'analyse de diverses cassettes à flux latéral comme les cassettes de test.

13. Dispositif selon l'une quelconque des revendications 1 à 12, conçu pour être utilisé avec un téléphone cellulaire pour fournir au moins l'un dudit élément de formation d'image et dudit processeur ; et
dans lequel de préférence les signaux audio des canaux gauche et droit du téléphone cellulaire sont conçus pour allumer et éteindre la DEL de réflexion et la DEL fluorescente.

14. Dispositif selon l'une quelconque des revendications 1 à 12, dans lequel l'élément de formation d'image comprend une ou plusieurs têtes de balayage.

15. Système pour l'analyse de divers types de cassettes pour des tests de diagnostic rapide par immunoessais, le système comprenant :
une ou plusieurs parois définissant une chambre fermée qui est conçue pour recevoir sélectivement les différentes cassettes une à la fois, chacune sous forme de cassette chargée, en relation sélectivement amovible ;
deux ou plusieurs diodes électroluminescentes (DEL) à l'intérieur de la chambre, qui comprennent au moins une diode électroluminescente à réflexion (DEL) conçue pour éclairer la cassette chargée lorsqu'elle est allumée, et au moins une DEL fluorescente conçue pour faire fluorescer la cassette chargée lorsqu'elle est allumée ;
une base de données qui comprend au moins un protocole d'essai associé à chacune des différentes cassettes d'essai ;
un téléphone cellulaire reçu par les parois de la chambre, le téléphone cellulaire comprenant : une caméra à l'intérieur de la chambre qui capte automatiquement une première image de la cassette chargée lorsqu'au moins une des diodes est allumée ; et au moins un processeur qui, en référence à la première image et en communication avec la base de données, identifie automatiquement un dit protocole de test applicable pour la cassette chargée ;
dans lequel le processeur automatiquement, après incubation de la cassette chargée et en fonction du un dit protocole de test applicable :
(i) détermine si la première image capte un signal de test post-incubation provenant de la cassette chargée ; et dans le cas contraire
(ii) allume la DEL fluorescente pour générer le signal de test par émission, ou la DEL de réflexion pour générer le signal de test par réflexion, à partir de la cassette chargée et utilise l'élément de formation d'image pour capter une deuxième image de la cassette chargée et du signal de test ; et dans lequel le processeur fournit automatiquement le signal de test, dans la première image ou la deuxième image, pour l'analyse de la cassette chargée ; et
dans lequel de préférence les signaux audio des canaux gauche et droit du téléphone cellulaire sont conçus pour allumer et éteindre la DEL de réflexion et la DEL fluorescente.

16. Procédé d'analyse de divers types de cassettes pour des tests de diagnostic rapide par immunoessais, le procédé comprenant :
une étape de réception consistant à recevoir de manière amovible les diverses cassettes une à la fois, chacune sous la forme d'une cassette chargée à l'intérieur d'une chambre fermée qui est définie par une ou plusieurs parois ;
une étape de diode électroluminescente (DEL) consistant à fournir deux ou plusieurs DEL à l'intérieur de la chambre, qui comprennent au moins une DEL de réflexion conçue pour éclairer la cassette chargée, et au moins une DEL fluorescente conçue pour faire fluorescer une zone de membrane de la cassette chargée ;
une étape de formation d'image consistant à capter automatiquement une première image de la cassette chargée, lorsqu'au moins une des DEL est allumée, en utilisant un élément de formation d'image à l'intérieur de la chambre ;
une étape de base de données consistant à fournir une base de données qui comprend au moins un protocole de test associé à chacune des différentes cassettes de test ; et
une étape de traitement dans laquelle au moins un processeur utilise la première image et la base de données pour identifier automatiquement un dit protocole de test applicable pour la cassette chargée ;
dans lequel, dans l'étape de traitement, le processeur automatiquement, après incubation de la cassette chargée et en fonction dudit protocole de test applicable :
(i) détermine si la première image capte un signal de test post-incubation provenant de la cassette chargée ; et dans le cas contraire
(ii) allume la DEL fluorescente pour générer le signal de test par émission, ou la DEL de réflexion pour générer le signal de test par réflexion, à partir de la cassette chargée et utilise l'élément de formation d'image pour capter une deuxième image de la cassette chargée et du signal de test ; et dans lequel, dans l'étape de traitement, le processeur fournit automatiquement le signal de test, dans la première image ou la deuxième image, pour l'analyse de la cassette chargée ; et de préférence
dans lequel, dans l'étape de traitement, le processeur analyse automatiquement le signal de test en référence à celui dudit protocole de test applicable pour la cassette chargée ; et encore plus préférablement
le signal de test comprend un signal de ligne de test correspondant à une ligne de test présente sur la cassette chargée après incubation, et le processeur mesure automatiquement une intensité du signal de ligne de test.

17. Procédé selon la revendication 16, dans lequel, dans l'étape de traitement, le signal de test comprend un signal de ligne de contrôle correspondant à une ligne de contrôle présente sur la cassette chargée après incubation, et le processeur mesure automatiquement une intensité du signal de ligne de contrôle ; et de préférence en plus ou en alternative, dans l'étape de traitement, ledit protocole de test applicable comprend une valeur de seuil d'essai prédéterminée, et le processeur analyse le signal de test en référence à la valeur de seuil d'essai pour déterminer automatiquement un résultat de test de diagnostic associé à la cassette chargée ; et encore plus préférablement
le procédé comprenant en outre une étape de présentation, dans laquelle le résultat du test de diagnostic est automatiquement présenté en utilisant un dispositif de sortie.

18. Procédé selon l'une quelconque des revendications 16 à 17, dans lequel, avant l'étape de traitement, un ou plusieurs ensembles d'instructions exécutables sont stockés dans une mémoire et, dans l'étape de traitement, les instructions exécutables codent le processeur pour analyser automatiquement le signal de test comme indiqué ci-dessus ; ou en variante
dans lequel, avant l'étape de traitement, un ou plusieurs ensembles d'instructions exécutables sont stockés dans une mémoire et, dans l'étape de traitement, les instructions exécutables codent le processeur pour identifier automatiquement celui applicable dudit protocole de test comme indiqué ci-dessus.

19. Procédé selon l'une quelconque des revendications 16 à 18, dans lequel, dans l'étape de traitement, le processeur utilise automatiquement un élément de communication pour référencer à distance la base de données comme indiqué ci-dessus.

20. Procédé selon l'une quelconque des revendications 16 à 19, comprenant en outre soit
une étape de filtrage consistant à fournir au moins un filtre optique, et dans lequel, dans l'étape de traitement, (i) le processeur s'assure automatiquement que le filtre optique est déplacé vers une position engagée entre la cassette chargée et l'élément de formation d'image, avant d'allumer une DEL d'émission correspondante, et l'élément de formation d'image capte alors le signal de test à travers le filtre optique, et (ii) le processeur s'assure automatiquement que le filtre optique est déplacé vers une position désengagée à l'écart de l'élément de formation d'image, avant d'allumer la DEL de réflexion, et l'élément de formation d'image capte alors le signal de test à l'écart du filtre optique ; ou
une étape de filtrage consistant à fournir un filtre optique passe-haut positionné entre la cassette chargée et l'élément de formation d'image, et dans lequel, dans l'étape de traitement, l'élément de formation d'image capte le signal de test à travers le filtre optique passe-haut.

21. Procédé selon l'une quelconque des revendications 16 à 20, dans lequel, dans l'étape de DEL, au moins une DEL blanche est fournie en tant que ladite au moins une DEL de réflexion ; et/ou
au moins une DEL fluorescente choisie parmi une DEL ultraviolette, une DEL colorée ou une combinaison de celles-ci est prévue.

22. Procédé selon l'une quelconque des revendications 16 à 21, dans lequel, dans l'étape de traitement, le processeur identifie ledit protocole de test applicable en référence à un ou plusieurs des éléments suivants qui sont saisis dans la première image, et stockés dans la base de données, pour la cassette chargée : une ou plusieurs dimensions de cassette, une ou plusieurs formes de cassette, une ou plusieurs dimensions de lignes de détection, une ou plusieurs dimensions de ligne de contrôle, une ou plusieurs zones de détection, une ou plusieurs zones de membrane, une ou plusieurs positions de ligne de contrôle, une ou plusieurs positions de ligne de test, une ou plusieurs couleurs de cassette, une ou plusieurs couleurs de ligne, des indications de fabricant, des indications de produit, des indications de marque, des indications d'application, des indications de maladie, des indications de type de test, des indications de temps d'incubation, des indications de résultats attendus, des codes à barres, des codes à barres bidimensionnels, des étiquettes et d'autres indications imprimées et écrites ; et de préférence en plus ou en alternative,
dans l'étape de traitement, le processeur identifie en outre ledit protocole de test applicable en référence à un ou plusieurs des éléments suivants qui sont reçus de la cassette chargée et stockés dans la base de données pour la cassette chargée : données stockées magnétiquement, données de fluorescence et données de signal radioactif.

23. Procédé selon l'une quelconque des revendications 16 à 22, dans lequel soit : dans au moins l'une de l'étape de formation d'image et de l'étape de traitement, un téléphone cellulaire est fourni en tant que dit élément de formation d'image et/ou en tant que dit processeur ; et de préférence
les signaux audio des canaux gauche et droit du téléphone cellulaire allument et éteignent la DEL de réflexion et la DEL fluorescente ; ou
dans l'étape de formation d'image, une ou plusieurs têtes de balayage sont prévues comme au moins une partie dudit élément de formation d'image.

24. Support lisible par ordinateur pour l'analyse de divers types de cassettes pour des tests de diagnostic rapide 2-P par immunoessais, configuré pour être utilisé avec le dispositif de la revendication 1 ;
avec le support lisible par ordinateur comprenant des instructions exécutables qui sont physiquement stockées sur celui-ci et qui, lors de l'exécution, codent au moins un processeur pour automatiquement :
capter une première image de la cassette chargée en utilisant l'élément de formation d'image lorsqu'au moins une des DEL est allumée ;
identifier, en utilisant la première image et la base de données, un dit protocole de test applicable pour la cassette chargée ;
après l'incubation de la cassette chargée et selon ledit protocole de test applicable :
(i) déterminer si la première image capte un signal de test post-incubation à partir de la cassette chargée ; et sinon (ii) allumer la DEL fluorescente pour générer le signal de test par émission, ou la DEL de réflexion pour générer le signal de test par réflexion, à partir de la cassette chargée et utiliser l'élément de formation d'image pour capter une seconde image de la cassette chargée et du signal de test ; et
fournir le signal de test, dans la première image ou la deuxième image, pour l'analyse de la cassette chargée.
